# EUROPEAN PATENT APPLICATION

(11) **EP 1 752 144 A1**
(43) Date of publication of application: **14.02.2007**
(21) Application number: 05743838.4
(22) Date of filing: 30.05.2005
(51) Int. Cl.: A61K 31/12, A23L 1/30, A61K 31/045, A61P 9/12

(54) **AGENT FOR PREVENTING/AMELIORATIG LIFE STYLE-RELAETD DISEASES CONTAINING TURMERIC ESSENTIAL OIL COMPONENT**

(30) Priority: 31.05.2004 JP 2004161590
(71) Applicant: Kaneka Corporation, Kita-ku Osaka-shi, Osaka 530-8288 (JP)
(72) Inventor: MAE, Tatsumasa, Kakogawa-shi, Hyogo 6750111 (JP); NISHIYAMA, Tozo, Akashi-shi, Hyogo 6730003 (JP); KISHIDA, Hideyuki, Kakogawa-shi, Hyogo 6750039 (JP); TAKAGAKI, Ryoji Maruzen Pharmaceuticals Co., Ltd., Fukuyama-shi, Hiroshima 7293102 (JP); YAMAMOTO, Masaji Maruzen Pharmaceuticals Co., Ltd., Fukuyama-shi, Hiroshima 7293102 (JP)
(74) Representative: HOFFMANN EITLE
(86) International application number: PCT/JP2005/009903
(87) International publication number: WO 2005/115366

(57) **Abstract**

The present invention has its object to provide an agent for preventing and/or ameliorating life style-related diseases which contains, as an active ingredient, a substance derived from a safe food material having a long history of being eaten as a food and which is capable of being utilized as functional foods such as health foods or functional health foods (specific health foods, functional nutritive foods).

The agent for preventing and/or ameliorating life style-related diseases according to the invention contains, as an active ingredient, at least one compound selected from the group consisting of ar-turmerone, α-turmerone, β-turmerone, curlone, bisacumol and β-sesquiphellandrene, or at least one compound selected from among the bisabolane type sesquiterpenoids derived from *Curcuma longa* L., and therefore is useful for preventing and/or ameliorating diabetes, visceral fat obesity, metabolic syndrome and obesity, among others.

## Description

### TECHNICAL FIELD

The present invention relates to an agent for preventing and/or ameliorating life style-related diseases which contains, as an active ingredient, an essential oil component derived from a plant material of the genus *Curcuma* origin, and a functional food containing the same.

### BACKGROUND ART

Life style-related diseases resulting from changes for the worse in life style, such as excessive nutrition and lack of exercise, are now a great social problem. Among such life style-related diseases, there may be mentioned obesity, diabetes, hyperlipidemia and hypertension, among others. A plurality of such morbid states may develop in combination and such combination is also termed metabolic syndrome, obesity, syndrome X, deadly quartet, insulin resistance syndrome or visceral fat syndrome, among others. The onset of metabolic syndrome is said to be based on insulin resistance and, further, it is said that there is the accumulation of visceral fat as a further upstream cause. Therefore, it is considered that such life style-related diseases as mentioned above may be prevented and/or alleviated by preventing and/or ameliorating the accumulation of visceral fat or the insulin resistance.

The peroxisome proliferator-activated receptor (PPAR) is a transcriptional regulatory factor serving to control the expression of a group of genes for maintaining the metabolism of sugars and lipids; it is a ligand-dependent transcriptional regulatory factor belonging to the nuclear receptor family. The PPAR includes three subtypes, namely PPARα, PPARγ and PPARδ. Among them, PPARγ is expressed in adipose tissues and is a master regulator controlling the differentiation and maturation of adipocytes. Such thiazolidine derivatives as troglitazone, pioglitazone and rosiglitazone developed as antidiabetics and agents for alleviating insulin resistance (insulin sensitizers) are PPARγ ligands activating PPARγ and showing a hypoglycemic activity and an insulin resistance-alleviating activity. It has been confirmed that these agents clinically reduce the visceral fat level; they are known to be effective not only against diabetes but also against life style-related diseases, typically metabolic syndrome.

Turmeric *(Curcuma longa* L.) is a perennial herb of the family Zingiberaceae, genus Zingiber and is generally known as turmeric, one of curry spices; it is used not only for food but also a coloring agent for food, clothing, etc. It is also used medicinally in Chinese medicine and in such traditional medicine as Ayurveda in India or Jamu in Indonesia owing to its hemostatic, stomachic, antibacterial and anti-inflammatory activities.

It is known that the main components of turmeric are yellow coloring matters curcuminoids, namely curcumin and its derivatives demethoxycurcumin and bisdemethoxycurcumin. While various physiological effects are known as the effects of turmeric or turmeric extracts, as mentioned above, most of the effects coincide with the physiological effects of the curcuminoids, in particular curcumin and, therefore, it is believed that the curcuminoids are principal active ingredients.

It is also known that turmeric contains essential oil components as well and most of them are bisabolane type sesquiterpenoids, for example ar-turmerone, α-turmerone and β-turmerone. Known physiological activities of turmeric essential oil component include mosquitocidal activity (cf. Non-Patent Document 1), apoptosis-inducing activity (cf. Non-Patent Document 2), prostaglandin and nitrogen oxide production-inhibiting activity (cf. Non-Patent Document 3 and 4) and liver function-improving activity.

On the other hand, among the plants of the family *Zingiberaceae,* genus *Zingiber (Curcuma* sp. ) , there are not only turmeric (autumn turmeric: *Curcuma long* L.) but also such varieties as wild turmeric (spring turmeric: *Curcuma aromatica* Salisb.), zedoary (purple turmeric: *Curcuma zedoaria* Rosc.) and xanthorrza *(Curcuma xanthorrhiza* Roxb.). These are herbs belonging to the same genus but differ in components contained therein. Thus, turmeric and xanthorrza are rich in the curcuminoids, typically curcumin, and all the species contain essential oil components but the compounds contained therein differ from species to species and are characteristic.

It has been disclosed that curcumenone (cf. Patent Document 1) and (4S,5S)-(+)-germacrone-4,5-epoxide (cf. Patent Document 2) contained in wild turmeric (spring turmeric: *Curcuma aromatica* Salisb.) have glucose tolerance improving activity and are useful as antidiabetics. Further, it has been disclosed that α-curcumene, a bisabolane type sesquiterpenoid contained in xanthorrza *(Curcuma xanthorrhizaRoxb.),* has serum triglyceride lowering activity and is useful as a lipid metabolism improving agent (cf. Patent Document 3). However, it is not known as yet that turmeric *(Curcuma longa* L.) -derived essential oil components, in particular the bisabolane type sesquiterpenoids ar-turmerone, α-turmerone and β-turmerone, have blood sugar lowering activity or blood sugar increase inhibiting activity, and visceral fat reducing activity.

Non-Patent Document 1: Roth, G. N. , et al., J. Nat. Prod., 61, 542-545, 1998

Non-Patent Document 2: Aratanechemuge, Y., et al., Int. J. Mol. Med., 9, 481-484, 2002

Non-Patent Document 3: Hong, C. H., et al., Planta Med., 68, 545-547, 2002

Non-Patent Document 4: Lee, S. K. et al., J. Environ. Pathol. Toxicol. Oncol., 21, 141-148, 2002

Patent Document 1: Japanese Kokai Publication Hei-01-233217

Patent Document 2: Japanese Kokai Publication Hei-06-192086

Patent Document 3: Japanese Kokai Publication Hei-07-149628

### SUMMARY OF THE INVENTION

It is an object of the present invention to provide an agent for preventing and/or ameliorating life style-related diseases which contains, as an active ingredient, a substance derived from a safe food material having a long history of being eaten as a food and which is capable of being utilized as functional foods such as health foods or functional health foods (specific health foods, functional nutritive foods).

The present inventors made intensive investigations to accomplish the above object and, as a result, found that a compound selected from among ar-turmerone, α-turmerone, β-turmerone, curlone, bisacumol and β-sesquiphellandrene, and the bisabolane type sesquiterpenoids derived from *Curcuma longa* L. has a blood sugar increase inhibiting activity and visceral fat reducing activity in an obesity-accompanied type II diabetes model and has PPARγ ligand activity.

The present invention has now been completed based on the above finding.

Thus, in a first aspect, the invention relates to
an agent for preventing and/or ameliorating life style-related diseases
which contains, as an active ingredient, at least one compound selected from the group consisting of ar-turmerone, α-turmerone, β-turmerone, curlone, bisacumol, and β-sesquiphellandrene.

The above-mentioned compound is preferably obtained from an essential oil component derived from a plant material of the genus *Curcuma* origin. In a second aspect, the invention relates to
an agent for preventing and/or ameliorating life style-related diseases
which contains, as an active ingredient, at least one compound selected from among the bisabolane type sesquiterpenoids derived from *Curcuma longa* L.

The life style-related disease so referred to with respect to the first aspect and second aspect of the invention includes diabetes, visceral fat obesity, metabolic syndrome and obesity, among others.

In a third aspect, the invention relates to
a PPAR ligand agent
which contains, as an active ingredient, at least one compound selected from the group consisting of ar-turmerone, α-turmerone, β-turmerone, curlone, bisacumol, and β-sesquiphellandrene.

The above-mentioned compound is preferably obtained from an essential oil component derived from a plant material of the genus *Curcuma* origin. In a fourth aspect, the invention relates to
a peroxisome proliferator-activated receptor ligand agent
which contains, as an active ingredient, at least one compound selected from among the bisabolane type sesquiterpenoids derived from *Curcuma longa* L.

The PPAR so referred to herein is PPARγ, for example.

### DETAILED DESCRIPTION OF THE INVENTION

In the following, the embodiments of the present invention will be described in detail.

The agent for preventing and/or ameliorating life style-related diseases according to the first aspect of the invention contains, as an active ingredient, at least one compound selected from the group consisting of ar-turmerone, α-turmerone, β-turmerone, curlone, bisacumol, and β-sesquiphellandrene. The agent for preventing and/or ameliorating life style-related diseases according to the second aspect of the invention contains, as an active ingredient, at least one compound selected from among the bisabolane type sesquiterpenoids derived from *Curcuma longa* L. The life style-related disease so referred herein includes diabetes, visceral fat obesity, metabolic syndrome and obesity, among others. The compound selected from among ar-turmerone, α-turmerone, β-turmerone, curlone, bisacumol and β-sesquiphellandrene, and the bisabolane type sesquiterpenoids derived from *Curcuma longa* L. has a blood sugar lowering activity, blood sugar increase inhibiting activity, and visceral fat reducing activity, and therefore is useful for preventing and/or ameliorating diabetes, and/or for preventing and/or ameliorating visceral fat obesity. Accordingly, the compound mentioned above is also useful for preventing and/or ameliorating such a life style-related disease as metabolic syndrome comprising two or more of diabetes (in particular type II diabetes), obesity (in particular visceral fat obesity), hyperlipidemia and hypertension, among others, or obesity.

The PPAR ligand agent, in particular the PPARγ ligand agent, according to the third aspect of the invention contains, as an active ingredient, at least one compound selected from the group consisting of ar-turmerone, α-turmerone, β-turmerone, curlone, bisacumol, and β-sesquiphellandrene. The PPAR ligand agent, in particular the PPARγ ligand agent, according to the fourth aspect of the invention contains, as an active ingredient, at least one compound selected from among the bisabolane type sesquiterpenoids derived from *Curcuma longa* L. The compound selected from among ar-turmerone, α-turmerone, β-turmerone, curlone, bisacumol and β-sesquiphellandrene, and the bisabolane type sesquiterpenoids derived from *Curcuma longa* L. activates PPARγ by binding to PPAR ligand-binding region, in particular PPARγ ligand-binding region, and therefore is useful for alleviating insulin resistance, and for preventing and/or ameliorating such a life style-related disease as metabolic syndrome comprising two or more of diabetes (in particular type II diabetes), obesity (in particular visceral fat obesity), hyperlipidemia and hypertension, among others, or obesity.

Turmeric is a safe food material having a long history of being eaten as a food. Twenty or more compounds are known as essential oil components derived from a plant material of the genus *Curcuma* origin. The ar-turmerone, α-turmerone, β-turmerone, curlone, bisacumol, and β-sesquiphellandrene, which are to be used in the present invention, are known as species of the bisabolane type sesquiterpenoids, which are essential oil components derived from turmeric (autumn turmeric: *Curcuma long* L.). The ar-turmerone, α-turmerone, β-turmerone, curlone, bisacumol, and β-sesquiphellandrene, which are to be used in the present invention, may be obtained from essential oil components derived from a plant material of the genus *Curcuma* origin, or chemically synthesized as long as they conform to food or food additive manufacturing standards, among others. Those obtained from an essential oil component derived from a plant material of the genus *Curcuma* origin are preferred. These 6 compounds may be used each as a single compound in the practice of the invention, or a mixture of two or more of them may also be used in the practice of the invention. As the compound mentioned above, ar-turmerone, α-turmerone and β-turmerone are preferred, and ar-turmerone is more preferred.

As the compound selected from among the bisabolane type sesquiterpenoids derived from *Curcuma longa* L., there may be mentioned, for example, ar-turmerone, α-turmerone and β-turmerone mentioned above, in addition curlone, bisacumol, and β-sesquiphellandrene. As the compound selected from among the bisabolane type sesquiterpenoids derived from *Curcuma longa* L., ar-turmerone, α-turmerone and β-turmerone are preferred, and ar-turmerone is more preferred.

The method for preparing the above-mentioned compounds to be used in the practice of the invention is not particularly restricted but any of those methods known in the art can be used. For example, an essential oil component can be obtained directly from a plant material of the genus *Curcuma* origin by such a method as solvent extraction using a hydrophobic solvent such as hexane, supercritical carbon dioxide extraction, or steam distillation. An essential oil component can also be obtained as a sesquiterpenoid fraction by subjecting a curcuma extract (extract, oleoresin) obtained by extraction with a solvent such as ethanol to column chromatography using silica gel or a resin for purification. The thus-obtained essential oil component derived from a plant material of the genus *Curcuma* origin generally contains about 50 to 60% by weight of a sum total of the bisabolane type sesquiterpenoids ar-turmerone, α-turmerone, β-turmerone, curlone, bisacumol, β-sesquiphellandrene. In addition, the method for obtaining such bisabolane type sesquiterpenoid compounds as ar-turmerone, α-turmerone and β-turmerone from the essential oil component derived from a plant material of the genus *Curcuma* origin is not particularly restricted but may comprise column chromatography using silica gel or a resin for purification, by which the compounds can be separated as a mixture or can be purified as respective single compounds.

The content of the compound mentioned above in the agent for preventing and/or ameliorating life style-related diseases according to the invention may be properly selected depending on the intended application but is preferably about 1 to 100% by weight and more preferably about 10 to 90% by weight. The agent for preventing and/or ameliorating life style-related diseases according to the invention may contain other ingredients for the purpose of improving the nutrition, taste, odor, flavor, property, etc. thereof.

When the an agent for preventing and/or ameliorating life style-related diseases according to the invention is taken for the above-mentioned compound(s) as an active ingredient (s) to produce the effects thereof, the total amount of the compound(s) per day per adult is desirably such that preferably about 0.1 to 1,000 mg/kg body weight, more preferably about 1 to 100 mg/kg body weight, be taken continuously.

The agent for preventing and/or ameliorating life style-related diseases of the invention can be utilized as or in functional foods such as health foods or functional health foods (specific health foods, functional nutritive foods). Such foods are not restricted in shape or form but the above agent can be utilized in supplement forms such as capsules and tablets; drink forms such as refreshing drinks and health drinks; or food forms such as processed foods and nutrient-adjusted foods. Such functional foods containing the agent for preventing and/or ameliorating life style-related diseases mentioned above also constitute an aspect of the present invention.

### (Effect of the invention)

According to the present invention, an agent for preventing and/or ameliorating life style-related diseases, which can be utilized as or in functional foods such as health foods or functional health foods (specific health foods, functional nutritive foods), may be provided. The agent for preventing and/or ameliorating life style-related diseases according to the invention contains, as an active ingredient, at least one compound selected from the group consisting of ar-turmerone, α-turmerone, β-turmerone, curlone, bisacumol, and β-sesquiphellandrene, or at least one compound selected from among the bisabolane type sesquiterpenoids derived from *Curcuma longa* L. The compound has a blood sugar increase inhibiting activity and visceral fat reducing activity, and therefore is useful for preventing and/or ameliorating diabetes, visceral fat obesity, metabolic syndrome and obesity, among others.

### BEST MODE FOR CARRYING OUT THE INVENTION

In the following, the present invention is described further in details by means of examples. However, these examples are no limitative of the present invention.

### (Example 1)

A turmeric powder (Kaneka Sun Spice Co., Ltd.; 700 g) was immersed in 3.5 liters of n-hexane, allowed to stand in the dark at room temperature for 3 days and then filtered to give a primary extract. The residue after filtration was immersed in 3.5 liters of n-hexane, allowed to stand in the dark at room temperature for 1 day and then filtered to give a secondary extract. The primary extract and secondary extract were combined and concentrated under reduced pressure to give 50.4 g of a hexane extract of turmeric.

As a result of silica gel thin-layer chromatography (TLC), it was confirmed that the hexane extract of turmeric contained essential oil components but did not contain any curcuminoids. The TLC was carried out using Silica Gel 60F₂₅₄ (Merck Ltd.) plates, with a 9:1 (v/v) chloroform-methanol mixture as a developing solvent.

### (Example 2)

The blood sugar increase inhibiting effect of the hexane extract of turmeric as prepared in Example 1 was evaluated using KK-A^{y} mice known as type II diabetes model animals.

KK-A^{y} mice (females, 6 weeks of age) were divided into two groups (5 animals per group), which were used as a control group and a hexane extract-dosed group. The control group was given a purified powder feed (Oriental Yeast Co.), and the hexane extract-dosed group was given the purified powder feed supplemented with 0.5% by weight of the hexane extract of turmeric as prepared in Example 1. The purified powder feed had the following composition: 20% by weight of casein, 49.948% by weight of corn starch, 10% by weight of sucrose, 10% by weight of soybean oil, 5% by weight of cellulose powder, 3.5% by weight of AIN-93 mineral mix, 1% by weight of AIN-93 vitamin mix, 0.25% by weight of choline bitartrate, 0.002% by weight of tert-butylhydroquinone and 0.3% by weight of L-cystine.

Small blood samples were collected from the mice via the caudal vein at the start of feeding and at 2 weeks and 4 weeks later. Each blood sample was measured for blood sugar using a Glutest Ace portable blood sugar meter (SANWA KAGAKU KENKYUSHO CO., LTD.). The results are shown in Table 1.

**Table 1**

| | Blood sugar level (mg/dl, mean±standard error, n=5) | |
|---|---|---|
| | Control group | Hexane extract group |
| Initial | 177±15 | 193 ± 13 |
| After 2 weeks | 360±36 | 262±17 (P<0.05) |
| After 4 weeks | 393±18 | 299±46 (P<0.1) |

In the control group, the blood sugar levels after 2 weeks and 4 weeks were higher as compared with the time of start of feeding, whereby it was confirmed that the animals became hyperglycemic. On the other hand, the sugar levels in the hexane extract-dosed group after 2 weeks and 4 weeks were clearly lower than those in the control group; thus, a blood sugar increase inhibiting effect was observed.

### (Example 3)

A turmeric oleoresin (Maruzen Pharmaceuticals Co., Ltd.; 30 g) was subjected to silica gel column chromatography, followed by elution with 10% (by volume) ethyl acetate/n-hexane. The eluate was concentrated to dryness to give 13.5 g of a sesquiterpenoid fraction of turmeric.

As a result of high-performance liquid chromatography (HPLC), it was confirmed that the sesquiterpenoid fraction of turmeric contained such bisabolane type sesquiterpenoids as ar-turmerone, α-turmerone and β-turmerone but did not contain any curcuminoids. The HPLC was carried out at 30°C using a TSKgel ODS-80Ts column (4.6 x 75 mm) (Tosoh Corporation) and an acetonitrile (A)-distilled water (B) system as the mobile phase under gradient conditions such that the concentration of A was increased from 45% to 70% at a constant rate from minute 0 to minute 15 and then maintained at 70% from minute 15 to minute 30. The flow rate was 0.7 ml/minute, the injection size was 20 µl, and the detection wavelength was 254 nm.

### (Example 4)

The blood sugar increase inhibiting effect and visceral fat reducing effect of the sesquiterpenoid fraction of turmeric as prepared in Example 3 was evaluated using KK-A^{y} mice known as type II diabetes model animals.

KK-A^{y} mice (females, 6 weeks of age) were divided into two groups (6 animals per group), which were used as a control group and a sesquiterpenoid fraction-dosed group. The control group was given a high-fat powder feed (Oriental Yeast Co.), and the sesquiterpenoid fraction-dosed group was given the high-fat powder feed supplemented with 0.24% by weight of the sesquiterpenoid fraction as prepared in Example 3. The high-fat powder feed had the following composition: 25% by weight of casein, 14.869% by weight of corn starch, 20% by weight of sucrose, 2% by weight of soybean oil, 14% by weight of lard, 14% by weight of tallow, 5% by weight of cellulose powder, 3.5% by weight of AIN-93 mineral mix, 1% by weight of AIN-93 vitamin mix, 0.25% by weight of choline bitartrate, 0.006% by weight of tert-butylhydroquinone and 0.375% by weight of L-cystine.

Small blood samples were collected from the mice via the caudal vein at the start of feeding and at 2 weeks and 4 weeks later. Each blood sample was measured for blood sugar using a Glutest Ace portable blood sugar meter (SANWA KAGAKU KENKYUSHO CO., LTD.). The results are shown in Table 2. After 5 weeks of feeding, the perirenal fat and the mesenteric fat within the abdominal cavity were excised from each mouse by anatomy and weighed. The results are shown in Table 3.

**Table 2**

| | Blood sugar level (mg/dl, mean±standard error, n=6) | |
|---|---|---|
| | Control group | Sesquiterpenoid fraction group |
| Initial | 155± 7 | 156± 6 |
| After 2 weeks | 389±27 | 309±16 (P<0.05) |
| After 4 weeks | 465±22 | 334±32 (P<0.01) |

**Table 3**

| | Weight of fat (g, mean ± standard error, n=6) | |
|---|---|---|
| | Control group | Sesquiterpenoid fraction group |
| Perirenal fat | 2.43±0.07 | 1.94+0.16 (P<0.05) |
| Mesenteric fat | 1.90±0.05 | 1.63±0.02 (P<0.01) |

In the control group, the blood sugar levels after 2 weeks and 4 weeks were higher as compared with the time of start of feeding, whereby it was confirmed that the animals became hyperglycemic. On the other hand, the sugar levels in the sesquiterpenoid fraction-dosed group after 2 weeks and 4 weeks were clearly lower than those in the control group; thus, a blood sugar increase inhibiting effect was observed.

The weights of the perirenal fat and the mesenteric fat in the sesquiterpenoid fraction-dosed group were clearly lower than in the control group; thus, a visceral fat reducing effect was observed.

### (Example 5)

A 3-g portion of the sesquiterpenoid fraction of turmeric prepared in Example 3 was subjected to ODS column chromatography, followed by elution with 65% (by volume) acetonitrile, whereby 0.7 g of ar-turmerone was isolated and purified. That the isolated and purified compound was ar-turmerone was confirmed by structural analysis by ¹H-NMR and ¹³C-NMR.

### (Example 6)

The hexane extract of turmeric as prepared in Example 1, the sesquiterpenoid fraction of turmeric as prepared in Example 3 and the ar-turmerone prepared in Example 5 were measured for PPARγ ligand activity levels.

CV-1 cells (male African green monkey kidney-derived cultured cells) were seeded onto a 96-well culture plate (6 x 10³ cells/well) and cultured under conditions of 37°C and 5% CO₂ for 24 hours. The medium used was DMEM (Dulbecco's modified Eagle medium; GIBCO) supplemented with 10% FBS (fetal bovine serum), 10 ml/L of a solution of penicillin and streptomycin (5,000 IU/ml and 5,000 µg/ml, respectively; GIBCO) and 37 mg/L of ascorbic acid (Wako Pure Chemical Industries, Ltd.). Cells were washed with OPTI-MEM (GIBCO), a serum-free medium for transfection, and then transfected with two plasmids, namely pM-PPARγ and 4xUASg-luc, using Lipofectamine Plus (Invitrogen Corporation), a gene transfer reagent. pM-PPARγ is a chimera protein expression plasmid resulting from joining of the yeast-derived transcription factor GAL4 gene (amino acid sequence 1-147) and the PPARγ ligand binding site gene (amino acid sequence 204-505), and 4xUASg-luc is a reporter plasmid with 4 repetitions of a GAL4 responsive element (UASg) as inserted upstream of the luciferase gene. At about 24 hours after transfection, the medium was replaced with a medium containing the sample (hexane extract of Example 1, sesquiterpenoid fraction of Example 3 or ar-turmerone of Example 5) (4 wells), followed by 24 hours of cultivation. Each sample was dissolved in dimethyl sulfoxide (DMSO) and the solution, or DMSO used in a no treatment control group, was added to the medium to each concentration given in Table 4. Cells were washed with phosphate-buffered saline (PBS+) containing Ca and Mg, then luclite (PerkinElmer), a luciferase chemiluminescence reagent, was added, and the luciferase-due chemiluminescence intensity was measured using a TopCount microplate scintillation/luminescence counter (PerkinElmer).

For each sample, the mean of luminescence intensities (4 wells) was determined, the ratio thereof to the value for the no treatment control was calculated and the relative activity was reported as the PPARγ ligand activity of the sample. The results obtained by carrying out the experiment in triplicate are shown in Table 4.

**Table 4**

| | Addition level | PPARγ ligand activity (mean±standard error, n=3) |
|---|---|---|
| No treatment control (DMSO) | (0.1%) | 1.00 |
| Positive control troglitazone | 0.5 µM | 2.10±0.31 |
| | 1 µM | 3.33±0.73 |
| Hexane extract | 5 µg/ml | 1.81±0.13 |
| | 10 µg/ml | 2.14±0.52 |
| Sesquiterpenoid fraction | 2.5 µg/ml | 1.79+0.54 |
| | 5 µg/ml | 2.30±0.84 |
| | 10 µg/ml | 2.49±0.55 |
| ar-turmerone | 2 µg/ml | 1.51±0.14 |
| | 5 µg/ml | 2.33+0.59 |

When troglitazone, a PPARγ ligand, was used as a positive control, a concentration-dependent PPARγ ligand activity was confirmed. Similarly, the hexane extract of turmeric, the sesquiterpenoid fraction and the ar-turmerone were found to have PPARγ ligand activity.

### (Example 7)

Using the sesquiterpenoid fraction of turmeric similarly prepared as in Example 3, a soft capsule was prepared by the common method according to the following composition.

| | |
|---|---|
| Sesquiterpenoid fraction | 40 parts by weight |
| Olive oil | 60 parts by weight |
| Vitamin E | 1 part by weight |

### INDUSTRIAL APPLICABILITY

According to the present invention, an agent for preventing and/or ameliorating life style-related diseases, which can be utilized as or in functional foods such as health foods or functional health foods (specific health foods, functional nutritive foods), may be provided. The agent for preventing and/or ameliorating life style-related diseases according to the invention contains, as an active ingredient, at least one compound selected from the group consisting of ar-turmerone, α-turmerone, β-turmerone, curlone, bisacumol, and β-sesquiphellandrene, or at least one compound selected from among the bisabolane type sesquiterpenoids derived from *Curcuma longa* L. The compound has a blood sugar increase inhibiting activity and visceral fat reducing activity, and therefore is useful for preventing and/or ameliorating diabetes, visceral fat obesity, metabolic syndrome and obesity, among others.

## Claims

1. An agent for preventing and/or ameliorating life style-related diseases
which contains, as an active ingredient, at least one compound selected from the group consisting of ar-turmerone, α-turmerone, β-turmerone, curlone, bisacumol, and β-sesquiphellandrene.

2. The agent for preventing and/or ameliorating life style-related diseases according to Claim 1
which contains, as an active ingredient, at least one compound selected from the group consisting of ar-turmerone, α-turmerone, and β-turmerone.

3. The agent for preventing and/or ameliorating life style-related diseases according to Claim 1 or 2
wherein the compound is obtained from an essential oil component derived from a plant material of the genus *Curcuma* origin.

4. An agent for preventing and/or ameliorating life style-related diseases
which contains, as an active ingredient, at least one compound selected from among the bisabolane type sesquiterpenoids derived from *Curcuma longa* L.

5. The agent for preventing and/or ameliorating life style-related diseases according to any one of Claims 1 to 4
wherein the life style-related disease is at least one species selected from the group consisting of diabetes, visceral fat obesity, metabolic syndrome and obesity.

6. The agent for preventing and/or ameliorating life style-related diseases according to any one of Claims 1 to 4
wherein the life style-related disease is diabetes.

7. The agent for preventing and/or ameliorating life style-related diseases according to any one of Claims 1 to 4
wherein the life style-related disease is visceral fat obesity.

8. The agent for preventing and/or ameliorating life style-related diseases according to any one of Claims 1 to 4
wherein the life style-related disease is metabolic syndrome or obesity.

9. A functional food which contains the agent for preventing and/or ameliorating life style-related diseases according to any one of Claims 1 to 8.

10. A peroxisome proliferator-activated receptor ligand agent
which contains, as an active ingredient, at least one compound selected from the group consisting of ar-turmerone, α-turmerone, β-turmerone, curlone, bisacumol, and β-sesquiphellandrene.

11. The peroxisome proliferator-activated receptor ligand agent according to Claim 10
which contains, as an active ingredient, at least one compound selected from the group consisting of ar-turmerone, α-turmerone, and β-turmerone.

12. The peroxisome proliferator-activated receptor ligand agent according to Claim 10 or 11
wherein the compound is obtained from an essential oil component derived from a plant material of the genus *Curcuma* origin.

13. A peroxisome proliferator-activated receptor ligand agent
which contains, as an active ingredient, at least one compound selected from among the bisabolane type sesquiterpenoids derived from *Curcuma longa* L.

14. The peroxisome proliferator-activated receptor ligand agent according to any one of Claims 10 to 13
wherein the peroxisome proliferator-activated receptor is a peroxisome proliferator-activated receptor γ.
